# EUROPEAN PATENT APPLICATION

(11) **EP 1 308 146 A1**
(43) Date of publication of application: **07.05.2003**
(21) Application number: 02024170.9
(22) Date of filing: 30.10.2002
(51) Int. Cl.: A61F 5/44, A61F 5/451, A61J 1/00, A61L 2/00, A61B 19/02

(54) **Sterilisable device for draining urine**

(30) Priority: 31.10.2001 IT BO20010091 U
(71) Applicant: Gallini S.r.l., 46100 Mantova (IT)
(72) Inventor: Avaltroni, Paolo, 46100 Mantova (IT)
(74) Representative: Dall'Olio, Giancarlo

(57) **Abstract**

A sterilisable device for draining urine includes a collecting bag (1) sealed until the moment in which the catheter is to be used. The bag (1), in turn, includes an elongated neck-like portion (3) and a collecting portion (5), joined to the elongated portion (3) and designed for collection of urine drained by the catheter (4). The catheter (4) is packed inside the collecting bag (1) in the region corresponding to the elongated portion (3), and can be extracted therefrom when the device (100) is to be used. The elongated portion (3) has an opening (6) at its distal end for allowing extraction of the catheter (4). The device includes also a segment (10) of liquid-impermeable material removably fastened to the elongated portion (3) at its distal end, so as to seal the opening (6).

## Description

The present invention relates to the manufacture of paramedical products and, in particular, of devices for draining urine.

In detail, the invention concerns a new disposable sterilisable device for draining urine, the device including a collection bag and a catheter.

It is known that catheters, in particular disposable catheters, which are used to drain urine, are usually packed within flexible bags. The bags are then airtightly sealed by heat-welding or high frequency-welding. The bags are so shaped as to form an elongated neck portion, designed to retain a catheter thereinside, joined to a larger portion, designed to collect the urine drained by means of the catheter. At the distal end of the neck portion, in the region corresponding to the tip portion of the catheter, there is a small slit, which forms a rupture-facilitated section.

Inside the collection portion, there is also a small container filled with sterile water. Also the small container is tightly closed. The container is usually formed from hard plastic material and features a precut line, which makes it easier to detach a part of the container. In this way, the liquid inside the container is released.

When the catheter is to be used, first the container inside the bag must be opened to let the water contained therein expand in the bag. As soon as the water contacts the catheter, a lubricating substance, previously applied to its outer surface, becomes active to facilitate insertion of the catheter in its operative seat.

Then, the distal end of the collecting bag neck portion is ruptured by tearing it crosswise, with the help of the above-mentioned slit. The catheter is then extracted and a final part, which is flared, is engaged by the opening and remains inside the elongated portion of the bag. The catheter is used and then disposed in accordance to the usual ways, which are commonly known.

A drawback often encountered when the above-mentioned package is used, derives from the form of the distal end of the bag, which is sometime difficult to open or defective. This can be usually caused by either faulty making of the slit or by lack of skill. More commonly, this problem occurs because of the partial physical inability, typical of the common user of this type of device, who is often affected by difficulties in coordination of movements. In such cases, the bag can result to be difficult to rupture or can rip along a wrong line, for instance longitudinally, so that the bag becomes unusable.

It is an object of the present invention to provide a new sterilisable device for draining urine, which device overcomes all the drawbacks mentioned above.

Another object of the invention is to provide a device for draining urine, which involves low costs for production and, consequently, also for sale.

The above mentioned objects are achieved by the features of the independent claim, while preferred features are defined in the dependent claims.

The invention will now be described in more detail with reference to particular, non-limiting embodiments and with reference to the accompanying drawings, in which:
Figure 1 shows schematically a lateral view of the device for draining urine manufactured in accordance with the present invention;
Figure 2 shows an enlarged view of a collecting bag elongated portion distal end;
With reference to the above-mentioned Figures, reference numeral 100 generally indicates a sterilisable device for draining urine, which has been manufactured in accordance with the basic features of the present invention.

The device 100, in particular, includes a bag 1 obtained from two pieces or flaps of flexible plastic material, of the type commonly used for this kind of products, which are superimposed to each other and sealed. In turn, the bag includes an elongated portion 3, forming a neck-like portion, designed to removably retain a catheter 4, and a collecting portion 5, joined to the elongated portion 3, for collecting urine coming from the catheter when it is used.

Furthermore, a container 30 is situated inside the collecting portion 5 and is filled with a liquid aimed at activating a lubricating substance applied to the surface of the catheter. A precut line 31 is made in the container for obtaining an easier breakage of the container 30, to allow the liquid contained therein to go out.

The elongated portion 3 has, at its distal end, an opening 6. Furthermore, the distal end of the elongated portion 3 has an extension 7 originated by one of the flap making up the elongated portion, and extending in the longitudinal direction of that single flap of the elongated portion 3, beyond the above-mentioned opening 6.

There is also a border 7a made on the distal end of the elongated portion 3 and along the edge of the extension 7.

The device 100 also includes a segment 10 made from a liquid-tight material. The liquid-tightness condition should be maintained at least for a time period sufficient for the lubricating substance of the catheter 4 to become active. While this material must prevent passage of liquid, however, it can be permeated by gases, which are commonly used for sterilisation of the package 100, for instance ethylene oxide. The segment 10 is removably fastened, with its own edge 11 and preferably by means of high-frequency welding, to the distal end of the elongated portion 3 and to the extension 7, along the border 7a, so that the opening 4 is sealed.

A grip portion 11a of the segment 10, outer with reference to the edge 11, is not attached to the elongated portion 3 so as to allow the user to grip the segment and easily remove it.

The segment 10, in particular, is advantageously obtained from a special type of micro-porous paper material which is permeable to gases, which are commonly used for sterilisation in medical and para-medical field, of instance ethylene oxide. The material must remain permeable at least for a pre-selected time period. This kind of material is available on the marketplace and is indicated by various brands, which are well known by those skilled in the art.

The elongated portion 3 has, near the opening 6, a narrow section 8, which is capable of engaging and retaining a flared portion 4a of the catheter 4 during its use. In this way, the catheter is prevented from getting out through the opening 6.

Preparation and subsequent use of the draining device 100 as described above, are well evident and easy to conceive, and are described in the following on a general basis only.

While the catheter 4 is being packed inside the bag 1, the segment 10 is applied and fastened to the bag 1 itself, in its proper operative position prior to sterilisation of the bag contents. The sterilisation gas, usually ethylene oxide, is then blown inside the bag 1. The gas passes through the above-mentioned segment 10, which is permeable to the above-mentioned sterilisation gas.

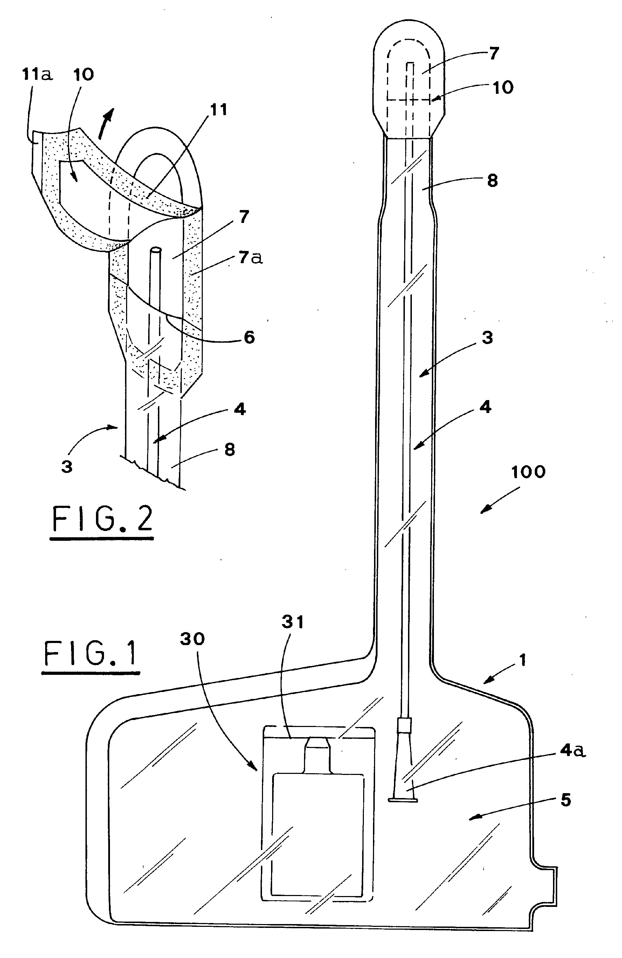

When the catheter 4 is used, the user or anybody who is performing the operation, must first break the container 30 along the precut line 31. Then, once the lubricating substance of the catheter 4 has become active, the segment 4 is removed by a simple operation including gripping the grip portion 11a and tearing the segment away, in the direction as indicated by the arrow in Figure 2.

The catheter 4 is then extracted up to the point in which the flared portion 4a is retained by the narrow section 8 of the elongated portion 3.

Lastly, usage of the catheter 4 and subsequent disposal of the draining device 100 are preformed in accordance with the usual ways.

The advantage deriving from the present invention basically include an easier opening operation of the draining device 100 and higher safety of the same operation, even when it is performed by unskilled people.

In this way a decrease of the production cost is obtained as well as, consequently, of the sale price.

## Claims

1. Sterilisable device for draining urine, including:
a collecting bag 1, comprising an elongated neck-like portion (3) and a collecting portion (5) joined to said elongated portion (3) and designed for collection of urine coming from a catheter (4), with an opening (6) made at the distal end of the elongated portion (3), said catheter being contained within said collecting bag (1) and situated, at least partially, within said elongated portion (3), such that said catheter (4) can be extracted through said opening (6) when the draining device (100) is used;
said sterilisable device being **characterised in that** it includes at least a segment (10) of liquid-impermeable material, removably fastened to the distal end of said elongated portion (3) such as to seal the opening (6) made therein.

2. Device according to claim 1, **characterized in that** said segment (10) is made from micro-porous paper capable of allowing passage of sterilising gas into said bag (1).

3. Device according to claim 2, **characterised in that** said bag includes two superimposed and sealed pieces of flexible plastic material and said elongated portion (3), at a distal end, has an extension (7) projecting from one piece of said of liquid-impermeable material beyond said opening (6), the extension (7) being provided with an enlarged border (7a) designed to match with a corresponding edge (11) of said segment (10).

4. Device according to claim 1, **characterized in that** said elongated portion (3) has, near said opening (6), a narrow section (8) for engaging and retaining a flared portion (4a) of said catheter (4).

5. Device according to claim 1, **characterized in that** said segment (10) is fastened to said elongated portion (3) by means of high-frequency welding.
